# EUROPÄISCHE PATENTSCHRIFT

(11) **EP 1 033 978 B1**
(45) Veröffentlichungstag und Bekanntmachung des Hinweises auf die Patenterteilung: **19.12.2001**
(21) Anmeldenummer: 99910359.1
(22) Anmeldetag: 18.03.1999
(51) Int. Cl.: A61K 9/70

(54) **D2-AGONIST ENTHALTENDES TRANSDERMALES THERAPEUTISCHES SYSTEM ZUR BEHANDLUNG DES PARKINSON-SYNDROMS UND VERFAHREN ZU SEINER HERSTELLUNG**
TRANSDERMAL THERAPEUTIC SYSTEM WHICH CONTAINS A D2 AGONIST AND WHICH IS PROVIDED FOR TREATING PARKINSONISM, AND A METHOD FOR THE PRODUCTION THEREOF
SYSTEME THERAPEUTIQUE TRANSDERMIQUE CONTENANT UN AGONISTE D2 SERVANT A TRAITER LE SYNDROME PARKINSONIEN, ET SON PROCEDE DE PRODUCTION

(30) Priorität: 30.03.1998 DE 19814084
(43) Veröffentlichungstag der Anmeldung: 13.09.2000
(73) Patentinhaber: LTS Lohmann Therapie-Systeme AG, 56626 Andernach (DE); Discovery Therapeutics, Inc., Richmond, Virginia 23230-3311 (US)
(72) Erfinder: MÜLLER, Walter, D-56564 Neuwied (DE); PECK, James, V., Richmond, VA 23233 (US)
(74) Vertreter: Flaccus, Rolf-Dieter, Dr.
(86) Internationale Anmeldenummer: EP9901795
(87) Internationale Veröffentlichungsnummer: WO9949852

(56) Entgegenhaltungen:
- WO-A-94/07468
- SWART, P. J. ET AL: "The influence of azone on the transdermal penetration of the dopamine D2 agonist N-0923 in freely moving rats" INT. J. PHARM. (1992), 88(1-3), 165-70 , XP002110532
- CHIANG, C. M. ET AL: "A two-phase matrix for the delivery of N-0923, a dopamine agonist" PROC. INT. SYMP. CONTROLLED RELEASE BIOACT. MATER. (1995), 22ND, 710-11 , XP002110533
- DEN DAAS, IZAAK ET AL: "Transdermal administration of the dopamine agonist N-0437 and seven ester prodrugs: comparison with oral administration in the 6-OHDA turning model" NAUNYN-SCHMIEDEBERG'S ARCH. PHARMACOL. (1990), 342(6), 655-9 , XP002110534

## Beschreibung

Die Erfindung bezieht sich auf ein transdermales therapeutisches System zur Behandlung des Parkinson-Syndroms mit einer gegenüber den Inhaltsstoffen der Matrix inerten Rückschicht, einer (-)-5,6,7,8-Tetrahydro-6-[propyl[2-(2-thienyl)ethyl]amino]-1-naphthol der nachstehenden Formel 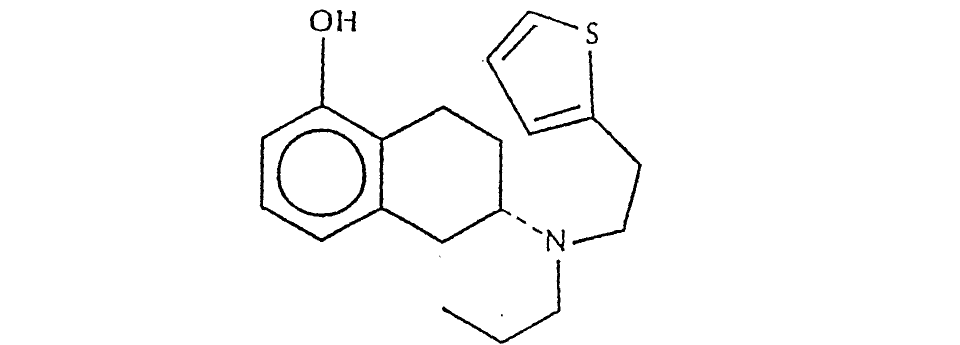 in wirksamer Menge aufweisenden selbstklebenden Matrixschicht und einer vor Gebrauch zu entfernenden Schutzfolie.

Weltweit leiden etwa 2,5 - 3% der Bevölkerung an dem sogenannten Parkinson-Syndrom, das hauptsächlich im Alter zwischen 58 und 62 Jahren zum Ausbruch kommt. Die Symptome dieser Krankheit äußern sich in motorischen Störungen wie Zittern und Muskelversteifung, vegetativen Störungen wie vermehrtem Speichel- und Tränenfluß, gestörter Wärmeregulation, erniedrigtem Blutdruck und Funktionsstörungen von Blase und Darm, sowie in psychischen Störungen wie Entschlußlosigkeit und depressiver Verstimmung.

Verursacht wird das Parkinson-Syndrom durch die Degeneration von dopaminergen Neuronen in der Substantia Nigra.

Dadurch verarmen bestimmte Hirnregionen, insbesonders die Hirnstammganglien, an Dopamin. Das daraus resultierende gestörte Gleichgewicht der Neurotransmitter Acetylcholin und Dopamin ist dabei letztendlich für die Symptome der Krankheit verantwortlich. Ein Übergewicht von Acetylcholin ist dabei für die sogenannten Plus-Symptome, ein Mangel an Dopamin für die sogenannten Minussymptome verantwortlich.

Die Behandlung des Parkinson-Syndroms kann deshalb mit sogenannten Anticholinergika oder Levodopa erfolgen. Anticholinergika hemmen die cholinerge Neurotransmission und Levodopa passiert als Vorstufe des Dopamins die Blut-Hirn-Schranke und wird im Hirn in Dopamin umgewandelt.

Ein anderer Weg zur Therapie des Parkinson-Syndroms ist die Behandlung mit Dopaminrezeptoragonisten. Dopaminagonisten sind Substanzen, die, obwohl strukturell von Dopamin verschieden, an die gleichen Rezeptoren binden und eine dem Dopamin vergleichbare Wirkung auslösen. Dopaminrezeptoragonisten haben dabei aufgrund ihrer Molekularstruktur Eigenschaften, die es ihnen ermöglichen, die Blut-Hirn-Schranke zu überwinden. Es ist dabei wegen der verminderten Nebenwirkungen vorteilhaft, wenn die Substanzen selektiv an eine Untergruppe der Dopaminrezeptoren, die D2 Rezeptoren, binden. Als besonders wirksamer selektiver D2 Agonist hat sich dabei die Substanz (-)-5,6,7,8-Tetrahydro-6-[propyl[2-(2-thienyl) ethyl]-amino]-1-naphthol mit der oben angegebenen Struktur ergeben.

Aufgrund ihrer kurzen Halbwertzeit und ihres hohen First-pass-Effekts ist jedoch die orale Verabreichung dieser Substanz sehr problematisch. Die kurze Halbwertzeit würde dabei eine oftmalige Einnahme der Substanz und der hohe First-pass-Effekt eine hohe Dosierung nötig machen. Während die Einnahmefrequenz durch eine geeignete orale Formulierung möglicherweise überwunden werden kann, ist das Problem des hohen First-pass-Effekts prinzipiell nur durch eine nicht orale Zufuhr des Wirkstoffs zu lösen.

Ein für die Verabreichung eines D2-Agonisten der oben genannten Formel vorgesehenes transdermales therapeutisches System wird bereits in der WO 94-07468 beschrieben. Dieses System enthält den Wirkstoff als Hydrochlorid in einer Zweiphasenmatrix, die im wesentlichen durch ein als durchgehende Phase vorliegendes hydrophobes Polymermaterial mit darin dispergiertem hydratisiertem Silikat zur Aufnahme des hydrophilen Arzneistoffsalzes gebildet wird und zusätzlich hydrophobe Lösungsmittel, permeationsfördernde und Dispergierungsmittel enthalten kann bzw. enthält.

Der Nachteil dieses Systems ist, daß das Wirkstoffsalz in wässriger Lösung mit dem Silikat gemischt werden muß, und ein zusätzlicher Emulgator notwendig ist, um diese wässrige Lösung mit dem in einem organischen Lösemittel - üblicherweise Hexan, Heptan oder Ethylacetat - gelösten lipophilen Polymer, z.B. einem Silikonkleber, zu emulgieren. Infolge von Beschichtungsproblemen ist es wesentlich schwieriger, transdermale Systeme unter Verwendung dieser Emulsion herzustellen. Zusätzlich kann für solche Systeme nur das Salz eingesetzt werden, da nur das Salz hydrophil genug ist, um in Wasser ausreichend löslich zu sein.

Aufgabe der Erfindung war es daher, Systeme für (-)-5,6,7,8-Tetrahydro-6-[propyl[2-(2-thienyl) ethyl]-amino]-1-naphthol zu entwickeln, die die Nachteile des in der WO 94-07468 beschriebenen Systems vermeiden. Dabei war insbesondere eine Optimierung von Wirkstoffaufnahme innerhalb des Systems und der Hauttransfer im Blickfeld.

Das demgemäß entwickelte erfindungsgemäße transdermale therapeutische System der eingangs genannten Art ist im wesentlichen
dadurch gekennzeichnet, dass die Matrixschicht
a) als Basis einen nicht-wäßrigen Polymerkleber auf Acrylat- bzw. Silikonbasis enthält,
b) eine Löslichkeit für die freie Base (-)-5,6,7,8-Tetrahydro-6-[propyl[2-(2-thienyl)-ethyl]amino]-1-naphthol von ≥ 5 % (g/g) besitzt, und
c) die freie Base (-)-5,6,7,8-Tetrahydro-6-[propyl[2-(2-thienyl)ethyl]amino]-1-naphthol in einer wirksamen Menge enthält.
Die Bestimmung der Löslichkeit erfolgt dabei bei Raumtemperatur.
Das transdermale system enthält optional weniger als 0,5 % (g/g), stärker bevorzugt weniger als 0,05 % (g/g) anorganische Silikatpartikel, oder die Matrix des transdermalen Systems ist im wesentlichen frei von anorganischen Silikatpartikeln.

Die Matrixsysteme stellen in ihrer einfachsten Ausführung eine Einphasenmatrix dar. Sie bestehen aus einer Rückschicht, einer wirkstoffhaltigen selbstklebenden Matrix und einer vor Gebrauch zu entfernenden Schutzfolie. Kompliziertere Ausführungen enthalten mehrschichtige Matrices, die auch nichtklebende Schichten und Steuermembranen enthalten können. Die Matrix kann wahlweise auch inerte Füllstoffe zur Verbesserung der Kohäsion enthalten.

Polyacrylate werden hergestellt durch radikalische Polymerisation von Acryl- bzw. Methacrylsäurederivaten, wobei durchaus auch andere geeignete Verbindungen wie z.B. Vinylacetat als zusätzliche Monomere eingesetzt werden können. Durch Auswahl der entsprechenden Monomeren können den resultierenden Klebern dabei jeweils spezifische Eigenschaften verliehen werden.

Üblicherweise werden Polyacrylate mit mehrwertigen Metallionen quervernetzt, um die physikalischen Eigenschaften des Klebers zu verbessern bzw. den jeweiligen Bedürfnissen anzupassen. Die Metallionen werden dabei meistens in der Form von in organischen Lösemitteln löslichen Metallchelaten eingesetzt. Geeignete Verbindungen sind dabei insbesondere Aluminiumacetylacetonat oder Titanacetylacetonat.

Silikonkleber stellen in den meisten Fällen Polydimethylsiloxane dar, allerdings können prinzipiell statt Methylgruppen auch andere organische Reste wie z.B. Ethyl- oder Phenylgruppen vorhanden sein. Es gibt solche Silikonkleber als Einkomponentenkleber in zwei Varianten, als sogenannte aminresistente und als nicht aminresistente Kleber. Aufgrund der basischen Natur von (-)-5,6,7,8-Tetrahydro-6-[propyl[2-(2-thienyl)ethyl]-amino]-1-naphthol werden für einen diesen Wirkstoff enthaltenden Silikonkleber aminresistente Kleber eingesetzt. Solche aminresistenten Silikonkleber zeichnen sich dadurch aus, daß sie über keine freien Silanolfunktionen verfügen. In einem speziellen Verfahren werden dabei die Si-OH-Gruppen mit einem Alkylrest versehen. Solche Kleber und ihre Herstellung sind in der EP 0 180 377 ausführlich beschrieben.

Das Lösevermögen der Kleber für den Wirkstoff ist ein für die Entwicklung von Matrixsystemen wichtiger Parameter, ebenso wie die Beweglichkeit des Wirkstoffs in der Matrix und sein Transfer über die Kontaktfläche hinweg zur Haut, der wesentlich durch entsprechende Verteilungskoeffizienten und die Hautresorption bestimmt wird. Es ergibt sich damit ein relativ kompliziertes Gefüge von Einflüssen, die zu berücksichtigen sind.

In Systemen, in denen der Wirkstoff nur zum Teil gelöst vorliegt, ist die Konzentration des gelösten Wirkstoffs gleich der Sättigungskonzentration und hat damit die unter diesen Bedingungen maximale thermodynamische Aktivität. Für das Lösevermögen der Polyacrylatkleber sind allgemein vor allem die Art und Menge der freien funktionellen Gruppen im Kleber wichtig. Bezüglich (-)-5,6,7,8-Tetrahydro-6-[propyl[2-(2-thienyl)ethyl)-amino]-1-naphthol wurde jedoch festgestellt, daß die Löslichkeit der freien Base davon weitgehend unabhängig ist und im Bereich von 15-35 % (g/g) liegt. Ein solches System muß deshalb den Wirkstoff in einer Konzentration von mindestens 10% (g/g) enthalten, um genügend nahe der maximalen thermodynamischen Aktivität zu sein. Für das Hydrochlorid von (-)-5,6,7,8-Tetrahydro-6-[propyl[2-(2-thienyl)ethyl]-amino]-1-naphthol liegt die Löslichkeit in Polyacrylaten mit 5-10% (g/g) wesentlich niedriger, so daß in solchen Systemen der Wirkstoff bevorzugt nur teilweise gelöst vorliegt.

Da das Hydrochlorid aufgrund seiner hydrophilen Eigenschaften nur schlecht die lipophile Barriere des Stratum Corneum passieren kann, ist in diesem Fall die Verwendung von lipophilen einwertigen Säuren wie z.B. Ölsäure notwendig, die in der Pflastermatrix das Hydrochlorid teilweise in das lipophilere Oleat überführt und darüber hinaus in der Haut generell als Permeationsenhancer wirkt.

Vorteilhaft enthält der Polymerkleber auf Acrylatbasis wenigstens zwei der folgenden Monomere:
Acrylsäure, Acrylamid, Hexylacrylat, 2-Ethylhexylacrylat, Hydroxyethylacrylat, Octylacrylat, Butylacrylat, Methylacrylat, Glycidylacrylat, Methacrylatsäure, Methacrylamid, Hexylmethylacrylat, 2-Ethylhexylmethacrylat, Octylmethacrylat, Methylmethacrylat, Glycidylmethacrylat, Vinylacetat, Vinylpyrrolidon.

Silikonkleber haben für die meisten Wirkstoffe ein vergleichsweise niedriges Lösevermögen. Die Sättigungskonzentration für die Base (-)-5,6,7,8-Tetrahydro-6-[propyl[2-(2-thienyl)ethyl]amino]-1-naphthol bzw. das Hydrochlorid liegt bei etwa 5 % (g/g), während die entsprechenden Salze darin praktisch unlöslich sind. In Verbindung mit Silikonklebern kommt deshalb nur die Wirkstoffbase in Frage. Ist dem Silikonkleber eine geeignete Substanz beigemischt, die über ein erhöhtes Lösevermögen für den Wirkstoff verfügt, kann die Löslichkeit für die freie Base in solchen Matrices auf bis zu 40 % (g/g) angehoben werden, ohne daß die physikalischen Eigenschaften der Matrix darunter leiden. Geeignete Substanzen sind z.B. lösliches Polyvinylpyrrolidon, Copolymere von Vinylpyrrolidon und Vinylacetat, Polyethylenglykol, Polypropylenglykol, Glycerin bzw. Fettsäureester von Glycerin oder Copolymere aus Ethylen und Vinylacetat, wobei sich Polyvinylpyrrolidon als besonders gut geeignet erwiesen hat.

Etwa 1,5-5 % (g/g) Polyvinylpyrrolidon in einem aminresistenten Silikonkleber erhöhen die Löslichkeit von (-)-5,6,7,8-Tetrahydro-6-[propyl[2-(2-thienyl)ethyl]-amino]-1-naphthol auf etwa 10-15 % (g/g). Dies ist ausreichend, um in einem 20 cm² großen Pflaster mit einem Beschichtungsgewicht der Matrix von 50 g/m² 10 mg Wirkstoff zu lösen. Da bei transdermalen Pflastersystemen immer davon ausgegangen werden muß, daß nur ca. 50% des eingesetzten Wirkstoffs während der Applikationszeit zur Verfügung stehen, kann bei einer Tagesdosis für den Wirkstoff im Bereich von etwa 1 - 10 mg davon ausgegangen werden, daß ein Pflaster in der Größe zwischen 2 und 40 cm² ausreichend ist, um therapeutische Plasmaspiegel zu erreichen.

Das in dem Silikonkleber dispergierte Polyvinylpyrrolidon hat dabei zusätzlich den Vorteil, daß es den bei Silikonklebern bekannten sogenannten kalten Fluß vermindert. Unter kaltem Fluß versteht man dabei, daß sich die Matrix wie eine sehr viskose Flüssigkeit verhält und entsprechend dazu neigt, durch Fließen eine größere Fläche einzunehmen. Das hat zur Folge, daß die Matrix nach einer gewissen Zeit eine größere Fläche als die Rückschicht des Pflasters einnimmt, und das Pflaster dazu neigt, mit dem Primärpackmaterial zu verkleben. Dieser Vorteil des Polyvinylpyrrolidons ist schon in der EP 0 524 776 erwähnt.

Zur Herstellung der Pflaster im Sinne dieser Erfindung wird (-)-5,6,7,8-Tetrahydro-6-[propyl[2-(2-thienyl)-ethyl]amino]-1-naphthol bzw. das Hydrochlorid in Ethanol oder einem anderen geeigneten organischen Lösemittel gelöst oder suspendiert und dann der Kleberlösung unter Rühren zugegeben. Besitzt der Kleber ein geeignetes Lösemittelsystem, kann der Wirkstoff auch direkt der Kleberlösung zugegeben werden. Zusätzliche Hilfsstoffe können entweder der Kleberlösung, der Wirkstofflösung oder der wirkstoffhaltigen Kleberlösung zugegeben werden. Ein Hilfsstoff, der vorteilhaft direkt der Wirkstofflösung zugesetzt wird, ist z.B. eine alkalisch reagierende Substanz, die geeignet ist, das Wirkstoffhydrochlorid in die freie Wirkstoffbase zu überführen. Bevorzugt wird ein Alkalimetallhydroxid wie Natrium- oder Kaliumhydroxid oder ein Alkalimetallsilikat wie Natrium- oder Kaliumtri- oder -metasilikat als alkalische Substanz zugegeben. Nach der Umsetzung kann die Lösung optional filtriert werden, wobei die Reaktanden mit Ausnahme der Wirkstoffbase praktisch quantitativ entfernt werden. Diese Reaktanden sind bei Einsatz von Natrium- bzw. Kaliumhydroxid Natrium-bzw. Kaliumchlorid und bei Einsatz von Natrium- bzw. Kaliumsilikaten Natrium- bzw. Kaliumchlorid und polymeres Siliziumdioxid. Vor der Filtration kann die Wirkstofflösung zusätzlich noch mit Puffersubstanzen versetzt werden, um eventuelle Überschüsse der Hilfsbasen zu neutralisieren. Die resultierende wirkstoffhaltige Kleberlösung wird auf eine geeignete Folie beschichtet und die Lösemittel in einem Trockenprozeß entfernt. Danach wird die Rückschicht des Pflasters auf die weitgehend lösemittelfreie Matrixschicht laminiert und aus dem Gesamtlaminat die Pflaster ausgestanzt.

Die Permeationseigenschaften werden vorteilhaft durch Permeationsenhancer verbessert, die aus der Gruppe der Fettalkohole, Fettsäuren, Fettsäureester, Fettsäureamide, Glycerin oder seinen Fettsäureestern, N-Methylpyrrolidon, Terpenen wie Limonen, α-Pinen, α-Terpineol, Carvone, Carveol, Limonenoxid, Pinenoxid, 1,8- Eukalyptol ausgewählt werden können.

Einzelheiten der Herstellung und die mit den fertigen Pflastern erreichten Permeationsraten können den Beispielen und den Permeationsstudien entnommen werden. Die in den Beispielen 1 - 3 genannten Polyacrylatkleber sind dabei als Beispiele zu betrachten und können durch andere für den medizinischen Gebrauch geeignete Acrylatkleber ohne Probleme ersetzt werden.

Mit den fertigen Pflastern wurden Permeationsstudien unter Verwendung von Franz-Diffusionszellen und menschlicher Epidermis durchgeführt. Die Ergebnisse sind in Zeichnung 1 festgehalten. Es zeigt sich, daß alle Pflaster in der Lage sind, eine genügende Menge des Wirkstoffs durch die Haut systemisch zur Verfügung zu stellen. Die vorliegende Erfindung zeigt, daß mit den freien Basen die Wirkstoffabgabe deutlich besser ist als unter Verwendung der Salze. Es zeigt sich auch, daß die Pflaster auf Basis von Silikonklebern bei einem wesentlich geringeren Wirkstoffgehalt etwa die gleiche Menge Wirkstoff durch die Haut abgeben als die Systeme auf Basis von Polyacrylatklebern.

Mit den erfindungsgemäßen Systemen ist es also möglich, die nötige Tagesdosis des Dopaminagonisten der angegebenen Struktur mit einem Pflaster einer Größe von ca. 20 cm² durch die Haut transdermal zu verabreichen. Da die Pflaster einfach herzustellen sind, den Wirkstoff auf ihrer gesamten Matrixfläche an die Haut abgeben und sowohl für die Wirkstoffsalze als auch für die Wirkstoffbasen geeignet sind, stellen sie eine wesentliche Verbesserung gegenüber bekannten Systemen dar, wie sie in der WO 94/07468 beschrieben sind.

### Beispiel 1: Polyacrylatsystem mit (-)-5,6,7,8-Tetrahydro-6-[propyl[2-(2-thienyl)-ethyl]-amino]-1-naphthol

Zu 264 g einer Lösung eines Polyacrylatklebers mit einem Feststoffgehalt von 50 % werden 66 g einer 50 %igen Lösung von Eudragit E100 in Ethylacetat gegeben und nach Zugabe von 36 g Oleylalkohol die Masse durch Rühren homogenisiert.

Danach werden 89,65 g (-)-5,6,7,8-Tetrahydro-6-[propyl[2-(2-thienyl)ethyl]amino]-1-naphthol in 200 ml Methylethylketon gelöst und obiger Masse unter Rühren zugegeben. Nachdem die Masse homogenisiert ist, wird sie mit einer geeigneten Rakel auf eine silikonisierte Polyesterfolie beschichtet. Die Dicke des feuchten Films ist so bemessen, daß nach dem Entfernen der Lösemittel durch 30-minütiges Trocknen bei 50 °C ein Beschichtungsgewicht von 60 g/m² resultiert.

Der getrocknete Matrixfilm wird nun mit einer 13 µm dikken Polyesterfolie kaschiert. Aus dem resultierenden Pflasterlaminat werden nun die fertigen Pflaster in der gewünschten Größe ausgestanzt und in Packstoffbeutel verpackt.

Die Konzentration von (-)-5,6,7,8-Tetrahydro-6-[propyl[2-(2-thienyl)ethyl]amino]-1-naphthol in der Pflastermatrix ist 30,8 %. Geeignete Polyacrylatkleber sind z.B. Durotak 387-2051, Durotak 387-2287, Durotak 387-2353, Durotal 387-2516, alle von National Starch & Chemical.

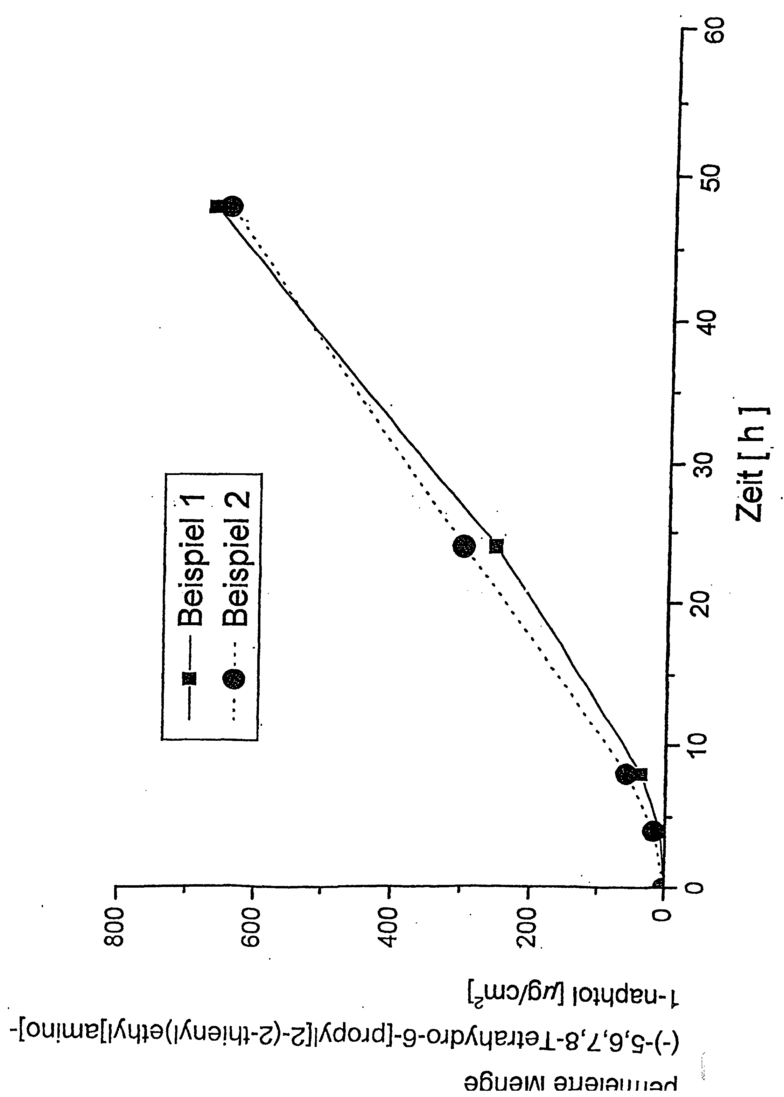

Die unter in-vitro Bedingungen erzielten Permeationsraten durch menschliche Epidermis sind in Fig. 1 dargestellt.

### Beispiel 2: Silikonsystem mit(-)-5,6,7,8-Tetrahydro-6-[propyl[2-(2-thienyl)-ethyl]-amino]-1-naphthol

Zu 24 g einer 25 prozentigen Lösung von Kollidon 90F werden 18 g (-)-5,6,7,8-Tetrahydro-6-[propyl[2-(2-thienyl)-ethyl]amino]-1-naphthol gelöst in 40 g Ethanol zugegeben und die Masse homogenisiert. Zu dieser Masse werden anschließend 251 g einer Lösung eines aminresistenten Silikonklebers mit einem Feststoffgehalt von 70 % gegeben und die Masse durch weiteres Rühren homogenisiert.

Anschließend wird die Masse mit einer geeigneten Rakel auf eine abhäsiv ausgerüstete Polyesterfolie (Scotchpak 1022) in der Dicke beschichtet, daß nach dem Entfernen der Lösemittel durch 30-minütiges Trocknen bei 50 °C ein Beschichtungsgewicht von 50 g/m² resultiert.

Der getrocknete Matrixfilm wird nun mit einer 13 µm dikken Polyesterfolie kaschiert. Aus dem resultierenden Pflasterlaminat werden die fertigen Pflaster in der gewünschten Größe ausgestanzt und in Packstoffbeutel verpackt.

Die Konzentration von (-)-5,6,7,8-Tetrahydro-6-[propyl[2-(2-thienyl)ethyl]amino]-1-naphthol Base in der Pflastermatrix ist 9 %.

Geeignete aminresistente Silikonkleber sind z.B. BIO-PSA Q7-4301 und BIO-PSA Q7-4201, beide von Dow Corning.

Die unter in-vitro Bedingungen erzielten Permeationsraten durch menschliche Epidermis sind in Fig.I dargestellt.

### Beispiel 3: Polyacrylatsystem mit dem Hydrochlorid von (-)-5,6,7,8-Tetrahydro-6-[propyl[2-(2-thienyl)-ethyl]-amino]-1-naphthol

10 g des Hydrochlorids werden in 70 g Polyacrylatkleber (Durotak 387-2287, Feststoffgehalt 50%, National Starch & Chemical) eingearbeitet und anschließend 4 g Ölsäure zugegeben. Die Masse wird nun auf eine silikonisierte Polyesterfolie in einer Dicke beschichtet, daß nach dem Entfernen der Lösemittel ein Beschichtungsgewicht von 60 g/m² resultiert. Die Lösemittel werden durch 15 - 20 minütiges Trocknen bei einer Temperatur zwischen 40 und 80°C entfernt. Danach wird die getrocknete Matrixschicht mit einer 12 - 30 µm dicken Polyesterfolie laminiert und die Pflaster ausgestanzt.

### BEISPIEL 4:

20 g (-)-5,6,7,8-Tetrahydro-6-[propyl[2-(2-thienyl)-ethyl]amino]-1-naphthol-hydrochlorid werden zusammen mit 8,0 g Natriummetasilikat oder 9,1 g Natriumsilikat in 35 ml Ethanol über 48 Stunden bei Raumtemperatur gerührt. Optional wird die Werkstofflösung nun filtriert und 6,0 g Polyvinylpyrrolidon (Kollidon F90, Fa. Bayer) in Form einer 25%igen (g/g) Lösung in Ethanol und 25 g einer 70%igen Lösung eines aminresistenten Silikonklebers (Q7-4301, Fa. Dow Corning) in Heptan zugegeben und die Masse anschließend durch mechanisches Rühren homogenisiert.

Anschließend wird die Masse zur Herstellung der Pflastermatrix auf eine geeignete abhäsiv ausgerüstete Folie beschichtet und. die Lösemittel durch 20minütiges Trocknen bei 50 °C entfernt. Das Beschichtungsgewicht des getrockneten Matrixfilms liegt bei 50 g/m².

Der getrocknete Matrixfilm wird mit einer 23 µm dicken Polyesterfolie kaschiert. Aus dem Gesamtlaminat werden die einzelnen Pflaster gestanzt.
Wird die Wirkstofflösung filtriert, entspricht das fertige Pflaster in seiner Zusammensetzung dem Pflaster gemäß Beispiel 2.

### BEISPIEL 5:

25 g (-)-5,6,7,8-Tetrahydro-6-[propyl[2-(2-thienyl)-ethyl]amino]-1-naphthol-hydrochlorid werden zusammen mit 14,7 g Natriummetasilikat oder 16,8 g Natriumtrisilikat in 40 ml Ethanol über 48 Stunden bei Raumtemperatur gerührt. Optional wird die Wirkstofflösung nun filtriert und 9,2 g Oleylalkohol, 63,2 g einer 52%igen Lösung eines Polyacrylatklebers (Durotak 387-2287, Fa. National Starch & Chemical) und 22,8 g einer 40 % (g/g) Lösung von Eudragit E100 (Röhm-Pharma) zugegeben und die Masse anschließend durch mechanisches Rühren homogenisiert.

Anschließend wird die Masse zur Herstellung der Pflastermatrix auf eine geeignete abhäsiv ausgerüstete Folie beschichtet und die Lösemittel durch 20minütiges Trocknen bei 50 °C entfernt. Das Beschichtungsgewicht des getrockneten Matrixfilms liegt bei 80 g/m².

Der getrocknete Matrixfilm wird mit einer 23 µm dicken Polyesterfolie kaschiert. Aus dem Gesamtlaminat werden die einzelnen Pflaster gestanzt.

### BEISPIEL 6:

20 g (-)-5,6,7,8-Tetrahydro-6-[propyl[2-(2-thienyl)-ethyl]amino]-1-naphthol-hydrochlorid werden in eine ethanolische NaOH oder KOH-Lösung gegeben, die äquimolare Mengen an Base (2,27 g NaOH bzw. 3,19 g KOH) enthält. Bevorzugt hat die Lösung eine Konzentration von 1,5 mol/l. Die Umsetzung des Wirkstoffsalzes erfolgt innerhalb von Minuten wobei der größte Teil des gebildeten NaCl ausfällt und die Wirkstoffbase komplett in Lösung geht. Optional wird nun die Wirkstofflösung mit einer Pufferlösung versetzt, um eventuelle Überschüsse der Base zu beseitigen. Ebenfalls optional kann die Wirkstofflösung nun filtriert werden; es werden 6,0 g Polyvinylpyrrolidon (Kollidon F90, Fa. Bayer) in Form einer 25%igen (g/g) Lösung in Ethanol und 250 g einer 70%igen Lösung eines aminresistenten Silikonklebers (Q7-4301, Fa. Dow Corning) in Heptan zugegeben und die Masse anschließend durch mechanisches Rühren homogenisiert.

Anschließend wird die Masse zur Herstellung der Pflastermatrix auf eine geeignete, abhäsiv ausgerüstete Folie beschichtet und die Lösemittel durch 20minütigees Trocknen bei 50 °C entfernt. Das Beschichtungsgewicht des getrockneten Matrixfilms liegt bei 50 g/m².

Der getrocknete Matrixfilm wird kaschiert mit einer 23 µm dicken Polyesterfolie; aus dem Gesamtlaminat werden die einzelnen Pflaster gestanzt.
Wird die Wirkstofflösung filtriert, entspricht das fertige Pflaster in seiner Zusammensetzung dem Pflaster gemäß Beispiel 2.

### BEISPIEL 7:

Analog Beispiel 6 werden 25 g (-)-5,6,7,8-Tetrahydro-6-[propyl [2-(2-thienyl)ethyl]amino]-1-naphthol)-hydrochlorid mit 2,84 g NaOH bzw. 3,99 g KOH in ethanolischer Lösung umgesetzt. Die Wirkstofflösung wird wie in Beispiel 6 optional mit Puffer versetzt bzw. filtriert und anschließend 9,2 g Oleylalkohol, 63,2 g einer 52%igen Lösung eines Polyacrylatklebers (Durotak 387-2287, Fa. National Starch & Chemical) und 22,8 g einer 40 % (g/g) Lösung von Eudragit E100 (Röhm-Pharma) zugegeben und die Masse dann durch mechanisches Rühren homogenisiert.

Anschließend wird die Masse zur Herstellung der Pflastermatrix auf eine geeignete abhäsiv ausgerüstete Folie beschichtet und die Lösemittel durch 20minütiges Trocknen bei 50 °C entfernt. Das Beschichtungsgewicht des getrockneten Matrixfilms liegt bei 80 g/m².

Der getrocknete Matrixfilm wird kaschiert mit einer 23 µm dicken Polyesterfolie; aus dem Gesamtlaminat werden die einzelnen Pflaster gestanzt.

## Patentansprüche

1. Transdermales therapeutisches System enthaltend eine gegenüber den Inhaltsstoffen der Matrix inerte Rückschicht, eine (-)-5,6,7,8-Tetrahydro-6-[propyl[2-(2-thienyl)ethyl]amino]-1-naphthol enthaltende, selbstklebende Matrixschicht und eine vor Gebrauch zu entfernende Schutzfolie, **dadurch gekennzeichnet, dass** die Matrixschicht
a) als Basis einen nicht-wäßrigen Polymerkleber auf Acrylat- bzw. Silikonbasis enthält,
b) eine Löslichkeit für die freie Base (-)-5,6,7,8-Tetrahydro-6-[propyl[2-(2-thienyl)ethyl]amino]-1-naphthol von ≥ 5 % (g/g) besitzt, und
c) die freie Base (-)-5,6,7,8-Tetrahydro-6-[propyl[2-(2-thienyl)ethyl]amino]-1-naphthol in einer wirksamen Menge enthält.

2. Transdermales therapeutisches System nach Anspruch 1, **dadurch gekennzeichnet, dass** die Matrix < 0,5 % (g/g) anorganische Silikatpartikel enthält.

3. Transdermales therapeutisches System nach Anspruch 1, **dadurch gekennzeichnet, dass** die Matrix < 0,05 % (g/g) anorganische Silikatpartikel enthält.

4. Transdermales System nach Anspruch 1, in welchem der Polymerkleber auf Acrylatbasis wenigstens zwei der folgenden Monomere enthält:
Acrylsäure, Acrylamid, Hexylacrylat, 2-Ethylhexyl-acrylat, Hydroxyethylacrylat, octylacrylat, Butylacrylat, Methylacrylat, Glycidylacrylat, Methacrylatsäure, Methacrylamid, Hexylmethylacrylat, 2-Ethylhexylmethacrylat, Octylmethacrylat, Methylmethacrylat, Glycidylmethacrylat, Vinylacetat oder Vinylpyrrolidon.

5. Transdermales System nach Anspruch 1, in welchem der Polymerkleber auf Silikonbasis Zusatzstoffe zur Verbesserung der Löslichkeit von (-)-5,6,7,8-Tetrahydro-6-[propyl[2-(2-thienyl)ethyl]amino]-1-naphthol in Form von hydrophilen Polymeren oder Glycerin oder Glycerinderivaten enthält.

6. Transdermales System nach Anspruch 4 oder 5, in welchem (-)-5,6,7,8,-Tetrahydro-6-[propyl[ 2-(2-thienyl)ethyl] amino]-1-naphthol in dem Polymerkleber auf Acrylatbasis in einer Konzentration von 10 bis 35 % [g/g] oder in dem Polymerkleber auf Silikonbasis in einer Konzentration von 5 bis 40 % [g/g] enthalten ist.

7. Transdermales System nach Anspruch 6, welches Substanzen enthält, die die Permeation von (-)-5,6,7,8-Tetrahydro-6-[propyl[2-(2-thienyl)ethyl]amino]-1-naphthol in die menschliche Haut verbessern.

8. Transdermales System nach Anspruch 7, in welchem die permeationsfördernde Substanz aus der Gruppe der Fettalkohole, Fettsäuren, Fettsäureester, Fettsäureamide, Glycerin oder seinen Derivaten, N-Methylpyrrolidon, Terpenen oder Terpenderivaten ausgewählt ist.

9. Transdermales System nach Anspruch 8, in welcher die permeationsfördernde Substanz ölsäure oder Oleylalkohol ist.

10. Transdermales System nach Anspruch 5, in welchem das hydrophile Polymer Polyvinylpyrrolidon, ein Copolymer von Vinylpyrrolidon und Vinylacetat, Polyethylenglykol, Polypropylenglykol oder ein Copolymer von Ethylen und Vinylacetat ist.

11. Transdermales System nach Anspruch 10, in welchem das hydrophile Polymer lösliches Polyvinylpyrrolidon ist und in einer Konzentration von 1,5-5 %(g/g) in der wirkstoffhaltigen Matrixschicht enthalten ist.

12. Transdermales System nach Anspruch 1, in welchem die Matrix inerte Füllstoffe zur Verbesserung der Kohäsion enthält.

13. Verfahren zur Herstellung eines transdermalen therapeutischen Systems, umfassend die folgenden Verfahrensschritte:
i) Mischen einer Suspension aus (-)-5,6,7,8-Tetrahydro-6-[propyl[2-(2-thienyl)ethyl]amino]-1-naphthol-hydrochlorid in Ethanol mit einer alkalischen Verbindung in Ethanol zur Umsetzung des Hydrochlorids in die freie Base,
ii) gegebenenfalls Filtern der entstandenen Suspension,
iii) Zugeben von Polyvinylpyrrolidon und einer Kleberlösung und
iv) Trocknen des Produktes.

14. Verfahren nach Anspruch 13, bei dem als alkalische Verbindung Natrium- oder Kaliumhydroxid eingesetzt wird.

15. Verfahren nach Anspruch 13, bei dem als alkalische Verbindung Natrium- oder Kaliummetasilikat oder -trisilikat eingesetzt wird.

16. Verfahren nach Anspruch 13, in welchem vor der Trocknung des Produktes die Mischung so auf eine inerte Rückschicht oder Schutzfolie beschichtet wird, daß ein einheitlicher Film entsteht.

17. Produkt, hergestellt nach einem Verfahren nach einem der Ansprüche 13 bis 16.

## Claims

1. Transdermal therapeutical system comprising a backing layer which is inert to the ingredients of the matrix, a self-adhering matrix layer containing (-)-5,6,7,8-tetrahydro-6-[propyl[2-(2-thienyl)ethyl]amino]-1-naphthol, and a protective layer which is to be removed before application, **characterized in that** the matrix layer
a) contains as a basis a non-aqueous polymer adhesive based on acrylate or silicone,
b) has a solubility of ≥ 5% (per weight) for the free base (-)-5,6,7,8-tetrahydro-6-[propyl[2-(2-thienyl)ethyl]-amino]-1-naphthol, and
c) contains the free base (-)-5,6,7,8-tetrahydro-6-[propyl[2-(2-thienyl)ethyl]amino]-1-naphthol in an effective amount.

2. Transdermal therapeutical system in accordance with claim 1, **characterized in that** the matrix contains < 0.5 % (per weight) inorganic silicate particles.

3. Transdermal therapeutical system in accordance with claim 1, **characterized in that** the matrix contains < 0.05 % (per weight) inorganic silicate particles.

4. Transdermal system according to claim 1 in which the acrylate-based polymer adhesive contains at least two of the following monomers: acrylic acid, acrylamide, hexylacrylate, 2-ethylhexylacrylate, hydroxyethylacrylate, octylacrylate, butylacrylate, methylacrylate, glycidylacrylate, methacrylic acid, methacrylamide, hexylmethacrylate, 2-ethylhexylmethacrylate, octylmethacrylate, methylmethacrylate, glycidylmethacrylate, vinylacetate or vinylpyrrolidone.

5. Transdermal system according to claim 1 in which the silicone-based polymer adhesive includes additives to enhance the solubility of (-)-5,6,7,8-tetrahydro-6-[propyl-[2-(2-thienyl)ethyl]amino]-1-naphthalenol in the form of hydrophilic polymers or glycerol or glycerol derivatives.

6. Transdermal system according to claim 4 or 5 in which (-)-5,6,7,8-tetrahydro-6-[propyl-[2-(2-thienyl)ethyl]-amino]-1-naphthalenol is contained in the acrylate-based polymer adhesive in a concentration of from 10 to 35% (w/w), or in the silicone-based polymer adhesive in a concentration of from 5 to 40% (w/w).

7. Transdermal system according to claim 6 that contains substances that enhance the permeation of (-)-5,6,7,8-tetrahydro-6-[propyl-[2-(2-thienyl)ethyl]amino]-1-naphthalenol into the human skin.

8. Transdermal system according to claim 7 in which the permeation-enhancing substance is selected from the group of fatty alcohols, fatty acids, fatty acid esters, fatty acid amides, glycerol or its derivatives, N-methylpyrrolidone, terpenes or terpene derivatives.

9. Transdermal system according to claim 8 in which the permeation-enhancing substance is oleic acid or oleyl alcohol.

10. Transdermal system according to claim 5, in which the hydrophilic polymer is polyvinylpyrrolidone, a copolymer of vinylpyrrolidone and vinylacetate, polyethyleneglycol, polypropylene glycol or a copolymer of ethylene and vinylacetate.

11. Transdermal system according to claim 10 wherein the hydrophilic polymer is soluble polyvinylpyrrolidone being present in the active substance-containing matrix layer at a concentration of 1.5 - 5% (w/w).

12. Transdermal system according to claim 1 in which the matrix contains inert fillers to improve cohesion.

13. A process for preparing a transdermal therapeutic system, comprising the following steps:
i) mixing a suspension of (-)-5,6,7,8-tetrahydro-6-[propyl-[2-(2-thienyl)ethyl]amino]-1-naphthalenol hydrochloride in ethanol with an alkaline compound in ethanol to convert the hydrochloride into the free base,
ii) optionally filtering the resultant suspension,
iii) adding polyvinylpyrrolidone and a solution of an adhesive, and iv) drying the product.

14. A process according to claim 13 wherein, as alkaline compound, sodium hydroxide or potassium hydroxide are used.

15. A process according to claim 13 wherein, as alkaline compound, sodium metasilicate or potassium metasilicate, or sodium or potassium trisilicate are used.

16. The process of claim 13 wherein before drying the product, the mixture is spread on an inert backing layer or protective foil or sheet in such a manner as to produce a uniform film.

17. A product prepared by a process according to one of the claims 13 to 16.

## Revendications

1. Système thérapeutique transdermique contenant une couche de base inerte par rapport aux ingrédients de la matrice, une couche de matrice autocollante contenant un (-)-5,6,7,8-tétrahydro-6-[propyl[2-(2-thiényl)éthyl]-amino-1-naphtol et une couche de protection à enlever avant l'utilisation, **caractérisé en ce que** la couche de matrice
a) contient comme base une colle polymère non aqueuse à base d'acrylate ou de silicone,
b) présente une solubilité ≥ 5 % (g/g) pour la base libre (-)-5,6,7,8-tétrahydro-6-[propyl[2-(2-thiényl)éthyl]amino-1-naphtol et
c) contient la base libre (-)-5,6,7,8-tétrahydro-6-[propyl[2-(2-thiényl)éthyl]amino-1-naphtol en une quantité efficace.

2. Système thérapeutique transdermique selon la revendication 1, **caractérisé en ce que** la matrice contient < 0,5 % (g/g) de particules inorganiques de silicate.

3. Système thérapeutique transdermique selon la revendication 1, **caractérisé en ce que** la matrice contient < 0,05 % (g/g) de particules inorganiques de silicate.

4. Système transdermique selon la revendication 1, dans lequel la colle polymère à base d'acrylate contient au moins deux des monomères suivants :
acide acrylique, acrylamide, acrylate d'hexyle, acrylate de 2-éthylhexyle, acrylate d'hydroxyéthyle, acrylate d'octyle, acrylate de butyle, acrylate de méthyle, acrylate de glycidyle, acide méthacrylique, méthacrylamide, acrylate d'hexylméthyle, méthacrylate de 2-éthylhexyle, méthacrylate d'octyle, méthacrylate de méthyle, méthacrylate de glycidyle, acétate de vinyle ou vinylpyrrolidone.

5. Système transdermique selon la revendication 1, dans lequel la colle polymère à base de silicone contient des additifs pour améliorer la solubilité du (-)-5,6,7,8-tétrahydro-6-[propyl[2-(2-thiényl)éthyl]amino-1-naphtol sous forme de polymères hydrophiles ou de glycérine ou de dérivés de glycérine.

6. Système transdermique selon la revendication 4 ou 5, dans lequel le (-)-5,6,7,8-tétrahydro-6-[propyl[2-(2-thiényl)éthyl]amino-1-naphtol est contenu dans la colle polymère à base d'acrylate en une concentration de 10 à 35 % [g/g] ou dans la colle polymère à base de silicone en une concentration de 5 à 40 % [g/g].

7. Système transdermique selon la revendication 6, qui contient des substances qui améliorent la perméation du (-)-5,6,7,8-tétrahydro-6-[propyl[2-(2-thiényl)éthyl]amino-1-naphtol dans la peau d'un être humain.

8. Système transdermique selon la revendication 7, dans lequel la substance favorisant la perméation est choisie parmi le groupe des alcools gras, des acides gras, des esters d'acide gras, des amides d'acide gras, de la glycérine ou ses dérivés, de la N-méthylpyrrolidone, des terpènes ou des dérivés des terpènes.

9. Système transdermique selon la revendication 8, dans lequel la substance favorisant la perméation est l'acide oléique ou l'alcool oléique.

10. Système transdermique selon la revendication 5, dans lequel le polymère hydrophile est de la polyvinylpyrrolidone, un copolymère de vinylpyrrolidone et d'acétate de vinyle, du polyéthylèneglycol, du polypropylèneglycol ou un copolymère d'éthylène et d'acétate de vinyle.

11. Système transdermique selon la revendication 10, dans lequel le polymère hydrophile est de la polyvinylpyrrolidone soluble et est contenu en une concentration de 1,5 à 5 % (g/g) dans la couche de matrice contenant la substance active.

12. Système transdermique selon la revendication 1, dans lequel la matrice contient des charges inertes pour l'amélioration de la cohésion.

13. Procédé pour la fabrication d'un système thérapeutique transdermique, comprenant les étapes de procédé suivantes :
i) mélange d'une suspension de chlorhydrate de (-)-5,6,7,8-tétrahydro-6-[propyl[2-(2-thiényl)éthyl]amino-1-naphtol dans de l'éthanol avec un composé alcalin dans de l'éthanol pour la transformation du chlorhydrate en base libre,
ii) le cas échéant filtration de la suspension formée,
iii) addition de polyvinylpyrrolidone et d'une solution d'adhésif et
iv) séchage du produit.

14. Procédé selon la revendication 13, dans lequel on utilise de l'hydroxyde de sodium ou de l'hydroxyde de potassium comme composé alcalin.

15. Procédé selon la revendication 13, dans lequel on utilise du métasilicate de sodium ou du métasilicate de potassium ou du trisilicate de sodium ou du trisilicate de potassium comme composé alcalin.

16. Procédé selon la revendication 13, dans lequel le mélange est revêtu avant le séchage du produit sur une couche de base inerte ou un film de protection inerte de telle manière qu'il se forme un film uniforme.

17. Produit, préparé selon un procédé selon une quelconque des revendications 13 à 16.
